# EUROPÄISCHE PATENTANMELDUNG

(11) **EP 4 002 378 A1**
(43) Veröffentlichungstag der Anmeldung: **25.05.2022**
(21) Anmeldenummer: 20208032.1
(22) Anmeldetag: 17.11.2020
(51) Int. Cl.: G16H 10/60

(54) **VERFAHREN, RECHENEINHEIT UND COMPUTERPROGRAMM ZUM ÜBERMITTELN VON MEDIZINISCHEN DATEN UND/ODER DOKUMENTEN**

(71) Anmelder: reachtag GmbH, 10625 Berlin (DE)
(72) Erfinder: GAREIS, Benjamin, 10713 Berlin (DE); AKSUNGUR, Egemen, 10781 Berlin (DE)
(74) Vertreter: Schulz Junghans Patentanwälte PartGmbB

(57) **Zusammenfassung**

Die Erfindung betrifft ein Verfahren zum Übermitteln von medizinischen Daten und/oder Dokumenten, wobei Identifikationsdaten eines ersten Benutzers (1) automatisch von einer ersten Kommunikationsvorrichtung (10) empfangen werden, die empfangenen Identifikationsdaten automatisch mit gespeicherten Identifikationsdaten verglichen werden, und wobei bei Übereinstimmung der empfangenen Identifikationsdaten mit den gespeicherten Identifikationsdaten gespeicherte medizinische Daten und/oder Dokumente des ersten Benutzers (1) automatisch abgerufen werden, zumindest ein Teil der abgerufenen medizinischen Daten und/oder Dokumente automatisch an die erste Kommunikationsvorrichtung (10) übermittelt wird, und automatisch eine Benachrichtigung an eine zweite Kommunikationsvorrichtung (20) übermittelt wird.

Die Erfindung betrifft weiterhin eine Recheneinheit (30) sowie ein Computerprogramm zur Durchführung des Verfahrens.

## Beschreibung

Die Erfindung betrifft ein Verfahren zum Übermitteln von medizinischen Daten und/oder Dokumenten eines ersten Benutzers, sowie eine Recheneinheit zur Durchführung des Verfahrens und ein Computerprogramm umfassend Befehle, die bei der Ausführung des Programms durch einen Computer diesen veranlassen, das Verfahren auszuführen. Das Verfahren dient insbesondere dazu, medizinische Daten und/oder Dokumente eines Patienten, der einen medizinischen Notfall erlitten hat, schnell und sicher unter anderem an medizinisches Personal zu übermitteln, um eine optimale Notfallversorgung des Patienten sicherzustellen und ggf. relevante Informationen und Daten und/oder Dokumente zur Verfügung zu stellen.

Aus dem Stand der Technik sind verschiedene Verfahren zum automatischen Übermitteln von medizinischen Daten und Dokumenten bekannt.

So beschreibt z.B. die Patentschrift US 6,988,075 ein elektronisches Dokumentationssystem für medizinische Daten mit einem Server, der mit einem Netzwerk verbunden ist und einer mit dem Server verbundenen Datenbank. Benutzer, wie z.B. medizinisches Personal, können z.B. von einem Computer auf den Server zugreifen und nach Eingabe einer Patientenidentifizierung patientenspezifische medizinische Informationen abrufen.

Die US-Patentanmeldung 2015/0025902 offenbart ein Telemedizinverfahren, bei dem per Telefon Informationen zwischen einem Arzt und einem Patienten ausgetauscht werden. Dabei kann z.B. die Patientenidentität durch eine Sprachabfrage verifiziert werden.

Gemäß der Patentschrift US 10,204,704 werden in einem Verfahren zum sicheren Übertragen von medizinischen Daten zunächst medizinische Informationen eines Patienten sowie dessen biometrische Daten in einem Datenspeichergerät gespeichert. Durch Scannen der biometrischen Daten können im Notfall die gespeicherten medizinischen Informationen abgerufen werden.

Aus der Patentschrift US 5,772,585 ist weiterhin eine computergestützte Methode zum Verwalten von Patienteninformationen in einer medizinischen Datenbank bekannt. Bei dem Verfahren werden Patientendaten in einer Datenbank kompiliert und nach Passwortabfrage einem Benutzer angezeigt, wobei z.B. eine Allergiewarnung ausgegeben werden kann.

In der US-Patentanmeldung 2017/0161439 ist ein Verfahren beschrieben, wobei mit einem Mobilgerät anhand von Positionsdaten bestimmt wird, ob sich ein Patient in der Nähe eines Krankenhauses oder einer Arztpraxis aufhält, wobei nach einer Identifizierung mit biometrischen Daten des Patienten zusätzlich medizinische Daten automatisch an einen Arzt übertragen werden können.

Schließlich beschreibt die Patentanmeldung JP 000342544 ein System zur Übertragung von medizinischen Daten an einen Arzt. Dabei ist eine automatische Auswahleinheit vorgesehen, die aus einem Speicher bestimmte Ärzte auswählt, die in der Lage sind, den Patienten zu behandeln.

Die im Stand der Technik beschriebenen Verfahren verlangen jedoch entweder ein aktives Mitwirken des Patienten (z.B. das aktive Bereitstellen seiner Identifizierungsdaten) oder bergen Sicherheitsrisiken, da die medizinischen Daten mithilfe der biometrischen Daten des Patienten (z.B. Scan des Fingerabdrucks) prinzipiell nicht nur von behandelndem medizinischem Personal oder in Notsituationen von einem weiten Spektrum an Vertrauten sondern von jedermann abgerufen werden können.

Davon ausgehend besteht die Aufgabe der vorliegenden Erfindung darin, ein Verfahren zum Übermitteln von medizinischen Daten und/oder Dokumenten zur Verfügung zu stellen, das es durch das System autorisiertem medizinischem Personal erlaubt, auch ohne aktives Mitwirken des Patienten notwendige medizinische Daten und/oder Dokumente in sicherer Weise und unter Wahrung des Datenschutzes zu erhalten.

Die Aufgabe wird durch den Gegenstand der unabhängigen Ansprüche 1 (Verfahren), 14 (Recheneinheit) und 15 (Computerprogramm) gelöst. Vorteilhafte Weiterbildungen der Erfindung sind in den Unteransprüchen 2-13 angegeben und sind im Folgenden beschrieben.

Ein erster Aspekt der Erfindung betrifft ein Verfahren zum Übermitteln von elektronisch gespeicherten medizinischen Daten und/oder Dokumenten, wobei Identifikationsdaten eines ersten Benutzers automatisch von einer ersten Kommunikationsvorrichtung, insbesondere von einer Recheneinheit, empfangen werden und die empfangenen Identifikationsdaten automatisch mit gespeicherten Identifikationsdaten verglichen werden, wobei bei Übereinstimmung der empfangenen Identifikationsdaten mit den gespeicherten Identifikationsdaten gespeicherte medizinische Daten und/oder Dokumente und/oder Informationen des ersten Benutzers automatisch, insbesondere aus einer Datenbank, abgerufen werden, zumindest ein Teil der abgerufenen medizinischen Daten und/oder Dokumente automatisch an die erste Kommunikationsvorrichtung übermittelt wird und automatisch eine Benachrichtigung an eine zweite Kommunikationsvorrichtung übermittelt wird.

Bei dem ersten Benutzer handelt es sich insbesondere um einen Patienten, der einen Unfall oder anderen medizinischen Notfall, z.B. einen Herzinfarkt oder Schlaganfall, erlitten hat und somit nicht ansprechbar und/oder nicht handlungsfähig ist. Der erste Benutzer hat insbesondere seine medizinischen Daten und/oder Dokumente bei einem Dienstleister hinterlegt, welcher eine Recheneinheit zur Durchführung des erfindungsgemäßen Verfahrens betreibt und die medizinischen Daten und/oder Dokumente des ersten Benutzers, z.B. in einer Datenbank, speichert. Die Identifikationsdaten sind insbesondere so an oder auf einem persönlichen Gegenstand des ersten Benutzers hinterlegt, dass sie im Notfall von einem zweiten Benutzer, insbesondere von medizinischem Personal wie Ärzten, Krankenhauspersonal oder Rettungssanitätern, aufgefunden werden können. Z.B. können die Identifikationsdaten, insbesondere in Form einer Zeichenkette, z.B. einer mehrstelligen Nummer, auf einem Aufkleber verzeichnet sein, der auf einem persönlichen Gegenstand des ersten Benutzers aufgeklebt ist. Hierfür eignet sich z.B. die Krankenversicherungskarte oder das Mobiltelefon des ersten Benutzers. Insbesondere im Fall eines Aufklebers auf der Krankenversichertenkarte können die Identifikationsdaten auch aus einer Kombination der auf dem Aufkleber verzeichneten Nummer mit einem Teil der auf der Karte verzeichneten Informationen wie die Versicherungsnummer (z.B. der letzten 4 Stellen) bestehen. Alternativ dazu könnten die Identifikationsdaten auch in elektronischer Form auf einem mobilen Gerät, wie einem Mobiltelefon, des ersten Benutzers gespeichert sein, so dass sie von dem zweiten Benutzer abgerufen werden können, wenn dieser das Gerät in Besitz nimmt.

Die erste Kommunikationsvorrichtung wird insbesondere von einem zweiten Benutzer bedient, d.h., der zweite Benutzer verwendet die erste Kommunikationsvorrichtung, um die Identifikationsdaten des ersten Benutzers zu übermitteln, insbesondere an die von dem Dienstleister betriebene Recheneinheit. Bei der ersten Kommunikationsvorrichtung kann es sich z.B. um ein Festnetztelefon (insbesondere in einem Krankenhaus oder einer Arztpraxis) oder ein Mobiltelefon (des zweiten Benutzers, insbesondere eines Arztes oder Rettungssanitäters) handeln. In diesem Fall kann der zweite Benutzer die Identifikationsdaten des ersten Benutzers z.B. übermitteln, indem er eine Telefonnummer des Dienstleisters anruft und die Identifikationsdaten, z.B. in Form der mehrstelligen Identifikationsnummer, über ein Tastenfeld des Telefons eingibt oder alternativ dazu per Spracheingabe übermittelt. Die Telefonnummer des Dienstleisters kann z.B. zusätzlich zu den Identifikationsdaten auf dem Aufkleber verzeichnet sein, welcher auf einem persönlichen Gegenstand des ersten Benutzers, z.B. auf einer Krankenversicherungskarte, aufgeklebt ist. Dabei kann die Telefonnummer z.B. auch in kodierter Form, z.B. in Form eines Barcodes oder QR-Codes auf dem Aufkleber verzeichnet sein. Dieser Aufkleber kann weiterhin Hinweise aufweisen, dass im Notfall die Telefonnummer des Dienstleisters angerufen und die Identifikationsnummer eingegeben werden soll, um die medizinischen Daten und/oder Dokumente des ersten Benutzers abzurufen.

Alternativ dazu kann es sich bei der ersten Kommunikationsvorrichtung auch um einen Computer, z.B. einen Desktopcomputer, ein Notebook, ein Tablet oder Ähnliches handeln. In diesem Fall (aber z.B. auch im Fall, dass es sich bei der ersten Kommunikationsvorrichtung um ein Smartphone handelt) könnte der zweite Benutzer die Identifikationsdaten z.B. über ein webseitenbasiertes Interface oder über eine App eingeben.

Die Recheneinheit des Dienstleisters erhält die Identifikationsdaten des ersten Benutzers und gleicht diese mit gespeicherten Identifikationsdaten ab, die insbesondere auf einem Speichermedium der Recheneinheit oder einem externen Speichermedium, auf das die Recheneinheit Zugriff hat, hinterlegt sind. Die Speicherung der Identifikationsdaten erfolgt insbesondere vorab bei Anmeldung des ersten Benutzers bei dem Dienstleister, z.B. im Format einer Datenbank.

In dieser Datenbank (und auf dem besagten Speichermedium) sind insbesondere weiterhin medizinische Daten des ersten Benutzers gespeichert. Diese werden von der Recheneinheit nur dann automatisch aus dem Speicher abgerufen, wenn die übermittelten Identifikationsdaten des ersten Benutzers mit gespeicherten Identifikationsdaten übereinstimmen.

Anschließend wird zumindest ein Teil der gespeicherten medizinischen Daten und/oder Dokumente automatisch aus dem Speicher abgerufen und automatisch an die erste Kommunikationsvorrichtung übermittelt. Somit erhält der zweite Benutzer schnell wichtige medizinische Daten und/oder Dokumente des ersten Benutzers, z.B. seine Blutgruppe und Allergien gegen Medikamente, so dass der zweite Benutzer optimale Notfallmaßnahmen zur Versorgung des ersten Benutzers einleiten kann. Der zweite Benutzer erhält die wichtigsten medizinischen Daten z.B. als Sprachnachricht oder Textnachricht auf der ersten Kommunikationsvorrichtung, z.B. seinem Mobiltelefon.

Unter dem Begriff "Textnachricht" ist im Kontext der vorliegenden Beschreibung eine elektronisch übertragene Nachricht jeglicher Art zu verstehen, welche Daten in Form von Text übermittelt. Dabei kann es sich z.B. um eine von einem Mobiltelefon, Smartphone oder Tablet versendete Kurznachricht ("SMS", die durch den Mobilfunkanbieter direkt über das Mobilfunknetz oder z.B. durch einen Drittanbieter über eine entsprechende App übertragen wird) oder um eine E-Mail handeln.

Durch die persönlichen Identifikationsdaten und den Abgleich der Identifikationsdaten ist sichergestellt, dass die medizinischen Daten und/oder Dokumente nur dann an den zweiten Benutzer übermittelt werden, wenn wirklich ein Notfall des ersten Benutzers vorliegt oder dieser z.B. das System testet und dass nicht die falschen medizinischen Daten und/oder Dokumente übermittelt werden, was den Datenschutz des ersten Benutzers sicherstellt.

Nach dem erfindungsgemäßen Verfahren wird zumindest ein Teil der abgerufenen medizinischen Daten und/oder Dokumente automatisch an die erste Kommunikationsvorrichtung übermittelt. Das bedeutet, es können z.B. zunächst nur die wichtigsten medizinischen Daten und/oder Dokumente übermittelt werden, die zur Einleitung schneller Notfallmaßnahmen essentiell sind, während weitere medizinische Daten und/oder Dokumente, z.B. medizinische Dokumente wie Vorsorgevollmachten oder Patientenverfügungen erst nach einer weiteren Sicherheitsabfrage an die erste Kommunikationsvorrichtung übermittelt werden. Dies sichert eine optimale medizinische Versorgung des Patienten und schützt gleichzeitig dessen Privatsphäre.

Weiterhin wird nach dem erfindungsgemäßen Verfahren automatisch eine Benachrichtigung an eine zweite Kommunikationsvorrichtung übermittelt. Dadurch erhält insbesondere ein dritter Benutzer, der Zugriff auf die zweite Kommunikationsvorrichtung hat, schnell eine Nachricht über den Unfall oder anderweitigen medizinischen Notfall des ersten Benutzers. Bei dem dritten Benutzer handelt es sich insbesondere um einen Notfallkontakt des ersten Benutzers, den der erste Benutzer insbesondere bei Anmeldung bei dem Dienstleister als Kontaktperson benannt hat. Die Kontaktdaten des dritten Benutzers sind insbesondere ebenfalls auf dem Speichermedium hinterlegt, z.B. in einem Datenbankeintrag zu dem ersten Benutzer. Die zweite Kommunikationsvorrichtung kann z.B. ein Mobiltelefon des dritten Benutzers sein.

Aufgrund der automatischen Übermittlung der Benachrichtigung erhält insbesondere die Notfall-Kontaktperson des Patienten schnell Kenntnis von dem medizinischen Notfall, und zwar ohne dass das versorgende medizinische Personal erst die Kontaktdaten des Notfallkontakts ermitteln und diesen manuell kontaktieren muss, wofür gerade in Notfallsituationen oft die Zeit fehlt.

Gemäß einer Ausführungsform des Verfahrens werden nach Übermittlung einer Bestätigungsnachricht von der zweiten Kommunikationsvorrichtung, insbesondere an die Recheneinheit, weitere medizinische Daten und/oder Dokumente, insbesondere die vollständigen medizinischen Daten und/oder Dokumente, des ersten Benutzers automatisch, insbesondere von der Recheneinheit, an die erste Kommunikationsvorrichtung übermittelt.

Das heißt, es kann zunächst nur ein Teil der abgerufenen bzw. gespeicherten medizinischen Daten und/oder Dokumente an die erste Kommunikationsvorrichtung übermittelt werden, z.B. die wichtigsten medizinischen Daten, wie Blutgruppe und Allergien gegen Medikamente, die für die medizinische Erstversorgung des ersten Benutzers wichtig sind.

In einem zweiten Schritt werden dann insbesondere nach einer Sicherheitsabfrage an den dritten Benutzer, z.B. den Notfallkontakt, welche dieser durch Übermittlung der Bestätigungsnachricht von der zweiten Kommunikationsvorrichtung, z.B. seinem Mobiltelefon, bestätigt, weitere medizinische Daten und/oder Dokumente an die erste Kommunikationsvorrichtung übermittelt. Bei diesen weiteren medizinischen Daten und/oder Dokumente kann es sich z.B. um besonders datenschutzrelevante Dokumente wie Patientenverfügungen, Vorsorgevollmachten oder Organspendeausweise handeln.

Durch die zusätzliche Sicherheitsabfrage beim dritten Benutzer, insbesondere Notfallkontakt, wird insbesondere der Datenschutz des ersten Benutzers verbessert.

Gemäß einer weiteren Ausführungsform des Verfahrens wird bei Übereinstimmung der empfangenen Identifikationsdaten mit den gespeicherten Identifikationsdaten, insbesondere von der Recheneinheit, automatisch eine Textnachricht an die zweite Kommunikationsvorrichtung übermittelt, wobei die Textnachricht eine Aufforderung zu einer Bestätigungseingabe enthält, wobei bei Erhalt der Bestätigungseingabe die Bestätigungsnachricht automatisch von der zweiten Kommunikationsvorrichtung, insbesondere an die Recheneinheit, übermittelt wird, woraufhin die weiteren medizinischen Daten und/oder Dokumente des ersten Benutzers, insbesondere die vollständigen medizinischen Daten und/oder Dokumente des ersten Benutzers, an die erste Kommunikationsvorrichtung übermittelt werden.

Auf diese Weise erhält insbesondere der dritte Benutzer eine Information in Textform über den medizinischen Notfall des ersten Benutzers. Diese kann z.B. weitere Informationen enthalten, mit denen der dritte Benutzer sicherstellen kann, dass die Sicherheitsabfrage wirklich von dem Dienstleister stammt, und kann diese vor Bestätigung verifizieren, was die Datensicherheit weiter verbessert.

Gemäß einer weiteren Ausführungsform des Verfahrens werden nach Übermittlung der Bestätigungsnachricht, insbesondere von der zweiten Kommunikationsvorrichtung an die Recheneinheit, die weiteren medizinischen Daten und/oder Dokumente des ersten Benutzers, insbesondere die vollständigen medizinischen Daten und/oder Dokumente des ersten Benutzers, dadurch, insbesondere von der Recheneinheit, an die erste Kommunikationsvorrichtung übermittelt, dass, insbesondere von der Recheneinheit, automatisch eine Textnachricht an die erste Kommunikationsvorrichtung gesendet wird, wobei die Textnachricht einen Dateianhang mit mindestens einer Datei, welche die weiteren medizinischen Daten und/oder Dokumente enthält, oder einen Link zum Abrufen der weiteren medizinischen Daten und/oder Dokumente enthält.

Somit kann der zweite Benutzer, insbesondere das medizinische Personal, die weiteren medizinischen Daten und/oder Dokumente des ersten Benutzers, insbesondere des Patienten, auf einfache Weise abrufen.

Gemäß einer weiteren Ausführungsform des Verfahrens wird nach Übermittlung der Bestätigungsnachricht, insbesondere von der zweiten Kommunikationsvorrichtung an die Recheneinheit, mindestens eine weitere Nachricht (z.B. eine Textnachricht), insbesondere von der Recheneinheit, an die zweite Kommunikationsvorrichtung übermittelt, wobei insbesondere die mindestens eine weitere Nachricht die an die erste Kommunikationsvorrichtung übermittelten medizinischen Daten und/oder Dokumente und/oder Kontaktinformationen enthält, mittels derer eine Kommunikationsverbindung zu der ersten Kommunikationsvorrichtung herstellbar ist.

Die weitere Nachricht (oder Nachrichten) hat/haben insbesondere den Zweck, dass der dritte Benutzer, insbesondere der Notfallkontakt, eine Information über die an den zweiten Benutzer (insbesondere medizinisches Personal) übermittelten Daten und/oder Dokumente des ersten Benutzers (insbesondere Patienten) sowie die Kontaktinformationen enthält, so dass der dritte Benutzer den zweiten Benutzer für eventuelle Rückfragen kontaktieren kann.

Bei den Kontaktinformationen handelt es sich insbesondere nicht um die Kontaktdaten (z.B. die Telefonnummer) der ersten Kommunikationseinrichtung, sondern z.B. um Kontaktdaten, insbesondere eine Telefonnummer, des Dienstleisters und einen Eingabebefehl (z.B. eine Ziffer gefolgt von dem Zeichen "#"), durch dessen Eingabe der dritte Benutzer durch einen Anruf bei der Telefonnummer des Dienstleisters eine automatische Anrufweiterleitung an die erste Kommunikationsvorrichtung aktivieren kann.

Gemäß einer weiteren Ausführungsform ist die zweite Kommunikationsvorrichtung ein Mobiltelefon, wobei die weitere Nachricht eine Kurznachricht (SMS) ist, und wobei die Kontaktinformationen eine Telefonnummer (z.B. des Dienstleisters, nicht aber der ersten Kommunikationsvorrichtung) sowie einen Eingabebefehl aufweisen, wobei durch einen Anruf der Telefonnummer durch die zweite Kommunikationsvorrichtung und die Eingabe des Eingabebefehls eine automatische Anrufweiterleitung an die erste Kommunikationsvorrichtung aktiviert wird. In diesem Fall ist bei dem Dienstleister insbesondere bereits die Telefonnummer der zweiten Kommunikationsvorrichtung gespeichert. Dadurch kann der Dienstleister auf die Eingabe von Identifikationsdaten verzichten und sofort eine Anrufweiterleitung an die erste Kommunikationsvorrichtung initiieren.

Gemäß einer weiteren Ausführungsform ist die weitere Nachricht eine E-Mail, wobei die Kontaktinformationen eine Telefonnummer (z.B. des Dienstleisters, nicht aber der ersten Kommunikationsvorrichtung), einen Eingabebefehl sowie einen Hinweis aufweisen, dass Identifikationsdaten, insbesondere die Identifikationsdaten des ersten Benutzers, eingegeben werden müssen, um eine automatische Anrufweiterleitung an die erste Kommunikationsvorrichtung zu aktivieren, wobei nach Anruf der Telefonnummer und Eingabe des Eingabebefehls und der Identifikationsdaten eine automatische Anrufweiterleitung an die erste Kommunikationsvorrichtung aktiviert wird.

In diesem Fall kann der dritte Benutzer den Anruf bei dem Dienstleister von einer beliebigen Kommunikationsvorrichtung (z.B. einem beliebigen Festnetz- oder Mobiltelefon) tätigen. Um hier einem Missbrauch durch nicht-autorisierte Personen vorzubeugen, müssen zusätzlich die Identifikationsdaten (insbesondere diejenigen des ersten Benutzers, welche dem dritten Benutzer, insbesondere Notfallkontakt, bekannt sind) eingegeben werden.

Dabei wird insbesondere sowohl eine Kurznachricht als auch eine E-Mail mit den Kontaktinformationen an die zweite Kommunikationsvorrichtung übermittelt, so dass der dritte Benutzer den automatischen Rückruf wahlweise direkt von der zweiten Kommunikationsvorrichtung oder von einem anderen Gerät initiieren kann.

Gemäß einer weiteren Ausführungsform wird für den Fall, dass nach Übermittlung der Textnachricht mit der Aufforderung zu der Bestätigungseingabe an die zweite Kommunikationsvorrichtung innerhalb einer vordefinierten Zeitspanne (z.B. 30 Sekunden) keine Bestätigungseingabe erfolgt (und somit auch keine Bestätigungsnachricht von der zweiten Kommunikationsvorrichtung versendet wird), mindestens eine weitere Nachricht (z.B. eine Textnachricht), insbesondere von der Recheneinheit, an die zweite Kommunikationsvorrichtung übermittelt, wobei insbesondere die mindestens eine weitere Nachricht die an die erste Kommunikationsvorrichtung übermittelten medizinischen Daten und/oder Dokumente und/oder Kontaktinformationen enthält, mittels derer eine Kommunikationsverbindung zu der ersten Kommunikationsvorrichtung herstellbar ist.

Wird auf diese weitere Nachricht hin die Kommunikationsverbindung zwischen der zweiten Kommunikationsvorrichtung und der ersten Kommunikationsvorrichtung hergestellt, wird insbesondere die Textnachricht mit der Aufforderung zu der Bestätigungseingabe erneut an die zweite Kommunikationsvorrichtung gesendet.

Gemäß einer weiteren Ausführungsform des Verfahrens wird bei Übereinstimmung der empfangenen Identifikationsdaten mit den gespeicherten Identifikationsdaten, insbesondere von der Recheneinheit, automatisch eine Textnachricht an die zweite Kommunikationsvorrichtung übermittelt, wobei die Textnachricht eine Aufforderung zu einer Bestätigungseingabe enthält, wobei bei Erhalt der Bestätigungseingabe die Bestätigungsnachricht automatisch von der zweiten Kommunikationsvorrichtung, insbesondere an die Recheneinheit, übermittelt wird, woraufhin die weiteren medizinischen Daten und/oder Dokumente des ersten Benutzers, insbesondere die vollständigen medizinischen Daten und/oder Dokumente des ersten Benutzers, an die erste Kommunikationsvorrichtung übermittelt werden.

Gemäß einer weiteren Ausführungsform des Verfahrens wird bei Übereinstimmung der empfangenen Identifikationsdaten mit den gespeicherten Identifikationsdaten, insbesondere von der Recheneinheit, automatisch ein Sprachanruf oder Videoanruf zwischen der ersten Kommunikationsvorrichtung und der zweiten Kommunikationsvorrichtung initiiert.

Ein Sprach- oder Videoanruf zwischen dem zweiten Benutzer (insbesondere medizinischem Personal) und dem dritten Benutzer (insbesondere Notfallkontakt) hat insbesondere gegenüber einer Textnachricht den Vorteil, dass der dritte Benutzer Rückfragen stellen kann und die Authentizität der Nutzung des Systems besser überprüfen kann. Daher wird der dritte Benutzer nach Austausch über einen Sprach- oder Videoanruf eher bereit sein, schnell eine Bestätigung zur Übermittlung der weiteren bzw. der vollständigen medizinischen Daten und/oder Dokumente des ersten Benutzers zu senden.

Gemäß einer weiteren Ausführungsform wird nach Abschluss des Sprach- oder Videoanrufs eine Textnachricht mit einem Teil der medizinischen Daten und/oder Dokumente des ersten Benutzers an die erste Kommunikationsvorrichtung übermittelt.

Insbesondere wird für den Fall, dass der Sprachanruf oder Videoanruf zwischen der ersten Kommunikationsvorrichtung und der zweiten Kommunikationsvorrichtung zustande kommt, automatisch, insbesondere von der Recheneinheit, eine Textnachricht an die zweite Kommunikationsvorrichtung übermittelt, wobei die Textnachricht die Aufforderung zu der Bestätigungseingabe enthält, wobei bei Erhalt der Bestätigungseingabe die Bestätigungsnachricht automatisch, insbesondere von der zweiten Kommunikationsvorrichtung an die Recheneinheit, übermittelt wird, woraufhin die weiteren medizinischen Daten und/oder Dokumente des ersten Benutzers, insbesondere die vollständigen medizinischen Daten und/oder Dokumente des ersten Benutzers, automatisch, insbesondere von der Recheneinheit, an die erste Kommunikationsvorrichtung übermittelt werden.

Über diese Textnachricht kann der dritte Benutzer die Sicherheitsabfrage im Kontext der zuvor über den Sprach- oder Videoanruf mit dem zweiten Benutzer ausgetauschten Informationen bewerten und die Freigabe der medizinischen Daten und/oder Dokumente schnell und einfach bestätigen.

Gemäß einer weiteren Ausführungsform des Verfahrens wird für den Fall, dass der Sprachanruf oder Videoanruf zwischen der ersten Kommunikationsvorrichtung und der zweiten Kommunikationsvorrichtung nicht zustande kommt, insbesondere von der Recheneinheit, automatisch eine Textnachricht an die zweite Kommunikationsvorrichtung übermittelt, wobei die Textnachricht Kontaktinformationen enthält, mittels derer eine Kommunikationsverbindung zu der ersten Kommunikationsvorrichtung herstellbar ist. Die Kontaktinformationen umfassen insbesondere nicht die Kontaktdaten (z.B. die Telefonnummer) der ersten Kommunikationseinrichtung, sondern z.B. Kontaktdaten, insbesondere eine Telefonnummer, (insbesondere des Dienstleisters) und einen Eingabebefehl (z.B. eine Ziffer gefolgt von dem Zeichen "#"), durch dessen Eingabe z.B. der dritte Benutzer durch einen Anruf bei der Telefonnummer des Dienstleisters eine automatische Anrufweiterleitung an die erste Kommunikationsvorrichtung aktivieren kann.

Mit den Kontaktinformationen kann der dritte Benutzer den ersten Benutzer (insbesondere das medizinische Personal) auf einfache Weise kontaktieren, um Informationen auszutauschen und dann den Abruf der weiteren bzw. vollständigen medizinischen Daten und/oder Dokumente zu bestätigen.

Gemäß einer weiteren Ausführungsform ist die zweite Kommunikationsvorrichtung ein Mobiltelefon, wobei die nach Nicht-Zustandekommen des Sprachanrufs oder der Videoverbindung automatisch an die zweite Kommunikationsvorrichtung übermittelte Textnachricht eine Kurznachricht ist, und wobei die Kontaktinformationen eine Telefonnummer sowie einen Eingabebefehl aufweisen, wobei durch einen Anruf der Telefonnummer durch die zweite Kommunikationsvorrichtung und die Eingabe des Eingabebefehls eine automatische Anrufweiterleitung an die erste Kommunikationsvorrichtung aktiviert wird.

Gemäß einer weiteren Ausführungsform ist die nach Nicht-Zustandekommen des Sprachanrufs oder der Videoverbindung automatisch an die zweite Kommunikationsvorrichtung übermittelte Textnachricht eine E-Mail, wobei die Kontaktinformationen eine Telefonnummer, einen Eingabebefehl sowie einen Hinweis aufweisen, dass Identifikationsdaten, insbesondere die Identifikationsdaten des ersten Benutzers, eingegeben werden müssen, um eine automatische Anrufweiterleitung an die erste Kommunikationsvorrichtung zu aktivieren, wobei nach Anruf der Telefonnummer und Eingabe des Eingabebefehls und der Identifikationsdaten eine automatische Anrufweiterleitung an die erste Kommunikationsvorrichtung aktiviert wird

Dabei wird insbesondere sowohl eine Kurznachricht als auch eine E-Mail mit den Kontaktinformationen an die zweite Kommunikationsvorrichtung übermittelt, so dass der dritte Benutzer den automatischen Rückruf wahlweise direkt von der zweiten Kommunikationsvorrichtung oder von einem anderen Gerät initiieren kann.

Gemäß einer weiteren Ausführungsform des Verfahrens wird für den Fall, dass der Sprachanruf oder Videoanruf zwischen der ersten Kommunikationsvorrichtung und der zweiten Kommunikationsvorrichtung nicht zustande kommt, insbesondere von der Recheneinheit, automatisch eine Textnachricht an die erste Kommunikationsvorrichtung übermittelt, wobei die Textnachricht einen Teil der abgerufenen medizinischen Daten und/oder Dokumente des ersten Benutzers enthält.

Mit anderen Worten erhält der erste Benutzer einen Teil der medizinischen Daten und/oder Dokumente in Textform, insbesondere falls der Sprach- oder Videoanruf mit dem dritten Benutzer nicht zustande kommt. Bei diesem Teil der medizinischen Daten und/oder Dokumente handelt es sich insbesondere um wichtige Notfalldaten des ersten Benutzers, wie die Blutgruppe oder Allergien gegen Medikamente, welche für eine optimale Notfallversorgung auch ohne bestätigte Sicherheitsabfrage durch den dritten Benutzer vorhanden sein sollten.

Gemäß einer weiteren Ausführungsform des Verfahrens werden die Identifikationsdaten des ersten Benutzers mittels eines Sprachanrufs oder eines Videoanrufs von der ersten Kommunikationsvorrichtung, insbesondere durch die Recheneinheit, empfangen.

Dadurch kann der zweite Benutzer (insbesondere medizinisches Personal) die Identifikationsdaten auf besonders einfache und schnelle Weise, ohne Notwendigkeit einer speziellen Applikation (z.B. Smartphone-App) und sogar ohne Zugang zum Internet übermitteln, um die medizinischen Daten und/oder Dokumente des ersten Benutzers (insbesondere Patient) zu erhalten.

Gemäß einer weiteren Ausführungsform des Verfahrens werden die Identifikationsdaten des ersten Benutzers über eine Tastatur, eine Kamera, ein Mikrophon oder einen Fingerabdruckscanner der ersten Kommunikationsvorrichtung oder über eine Tastatur, eine Kamera, ein Mikrophon oder einen Fingerabdruckscanner eines separaten Eingabegeräts erfasst. Die über die Kamera erfassten Bilder können z.B. verwendet werden, um mit einem Gesichtserkennungsalgorithmus die Identität des ersten Benutzers zu ermitteln.

Gemäß einer weiteren Ausführungsform des Verfahrens wird zumindest der Teil der abgerufenen medizinischen Daten und/oder Dokumente mittels einer Sprachnachricht, insbesondere von der Recheneinheit, automatisch an die erste Kommunikationsvorrichtung übermittelt.

Auf diese Weise erhält der zweite Benutzer (insbesondere medizinisches Personal) die wichtigsten medizinischen Daten und/oder Dokumente auf möglichst schnelle und intuitive Weise.

Gemäß einer weiteren Ausführungsform des Verfahrens umfassen die medizinischen Daten und/oder Dokumente persönliche Daten des ersten Benutzers.

Gemäß einer weiteren Ausführungsform des Verfahrens umfassen die persönlichen Daten ein Geburtsdatum des ersten Benutzers, ein Geschlecht des ersten Benutzers, eine Körpergröße des ersten Benutzers, ein Körpergewicht des ersten Benutzers, Informationen über eine Augenfarbe des ersten Benutzers, Informationen über eine Blutgruppe des ersten Benutzers, Informationen über das Vorhandensein mindestens einer Allergie des ersten Benutzers, Informationen über das Vorhandensein mindestens einer Krankheit, z.B. einer chronischen Krankheit, des ersten Benutzers, Informationen über mindestens ein Medikament, welches der erste Benutzer einnimmt und/oder Informationen über mindestens eine an dem ersten Benutzer durchgeführte Operation umfassen.

Das Geburtsdatum des Patienten ist z.B. bei Kindern notwendig, um das Alter des Kindes zu ermitteln und sicherherzustellen, ob ein bestimmtes Medikament eingesetzt werden kann, das für Kinder eines bestimmten Alters zugelassen ist.

Körpergröße und Gewicht können vor allem bei der Ermittlung der korrekten Dosierung bestimmter Medikamente wichtig sein. Die Übermittlung dieser Daten ist vor allem bei zeitkritischen Therapien wichtig, um z.B. keine Zeit für eine Messung von Körpergröße und Gewicht zu verschwenden.

Die Blutgruppe des Patienten dient vor allem dazu, bei einer Bluttransfusion kompatibles Spenderblut auszusuchen.

Weiterhin sind Kenntnisse über Allergien gegen bestimmte Medikamente wichtig, um die richtigen Medikamente für eine Therapie auszusuchen und eine gefährliche allergische Reaktion des Patienten zu vermeiden.

Schließlich müssen auch Kenntnisse über Vorerkrankungen, bereits durchgeführte Operationen und eingenommene Medikamente bei der Planung der Therapie berücksichtigt werden.

Gemäß einer weiteren Ausführungsform des Verfahrens umfassen die medizinischen Daten und/oder Dokumente mindestens ein medizinisches Dokument des ersten Benutzers.

Gemäß einer weiteren Ausführungsform des Verfahrens ist das medizinische Dokument eine Vorsorgevollmacht, eine ärztliche Notfallanordnung, eine Patientenverfügung, ein Organspendeausweis, ein Impfpass und/oder ein Allergiepass.

Diese Dokumente sind insbesondere in einer übersichtlichen Form und in einem geeigneten Dateiformat durch den Dienstleister hinterlegt, z.B. in der Datenbank, und können mit dem erfindungsgemäßen Verfahren dem zweiten Benutzer (insbesondere dem medizinischen Personal) auf einfache Weise zugänglich gemacht werden.

Ein zweiter Aspekt der Erfindung betrifft eine Recheneinheit zum Übermitteln von medizinischen Daten und/oder Dokumenten, insbesondere zur Durchführung eines Verfahrens nach dem ersten Aspekt der Erfindung, wobei die Recheneinheit dazu konfiguriert ist, Identifikationsdaten eines ersten Benutzers von einer ersten Kommunikationsvorrichtung automatisch zu empfangen, die empfangenen Identifikationsdaten automatisch mit gespeicherten Identifikationsdaten zu vergleichen, und wobei die Recheneinheit weiterhin dazu konfiguriert ist, bei Übereinstimmung der empfangenen Identifikationsdaten mit den gespeicherten Identifikationsdaten automatisch gespeicherte medizinische Daten und/oder Dokumente des ersten Benutzers, insbesondere aus einer Datenbank, abzurufen, zumindest einen Teil der abgerufenen medizinischen Daten und/oder Dokumente automatisch an die erste Kommunikationsvorrichtung zu übermitteln und automatisch eine Benachrichtigung an eine zweite Kommunikationsvorrichtung, insbesondere eines dritten Benutzers, weiter insbesondere eines Notfallkontakts des ersten Benutzers, zu übermitteln.

Gemäß einer Ausführungsform ist die Recheneinheit dazu konfiguriert, nach Übermittlung einer Bestätigungsnachricht von der zweiten Kommunikationsvorrichtung weitere medizinische Daten und/oder Dokumente, insbesondere die vollständigen medizinischen Daten und/oder Dokumente, des ersten Benutzers automatisch an die erste Kommunikationsvorrichtung übermittelt werden.

Gemäß einer weiteren Ausführungsform ist die Recheneinheit dazu konfiguriert, bei Übereinstimmung der empfangenen Identifikationsdaten mit den gespeicherten Identifikationsdaten automatisch eine Textnachricht an die zweite Kommunikationsvorrichtung zu übermitteln, wobei die Textnachricht eine Aufforderung zu einer Bestätigungseingabe enthält, und wobei die Recheneinheit dazu konfiguriert ist, bei Erhalt der Bestätigungseingabe die Bestätigungsnachricht automatisch von der zweiten Kommunikationsvorrichtung zu empfangen.

Gemäß einer weiteren Ausführungsform ist die Recheneinheit dazu konfiguriert, nach Übermittlung der Bestätigungsnachricht die weiteren medizinischen Daten und/oder Dokumente des ersten Benutzers dadurch an die erste Kommunikationsvorrichtung zu übermitteln, dass automatisch eine Textnachricht an die erste Kommunikationsvorrichtung gesendet wird, wobei die Textnachricht einen Dateianhang mit mindestens einer Datei, welche die weiteren medizinischen Daten und/oder Dokumente enthält, oder einen Link zum Abrufen der weiteren medizinischen Daten und/oder Dokumente enthält.

Gemäß einer weiteren Ausführungsform ist die Recheneinheit dazu konfiguriert, nach Übermittlung der Bestätigungsnachricht mindestens eine weitere Nachricht an die zweite Kommunikationsvorrichtung zu übermitteln, wobei insbesondere die mindestens eine weitere Nachricht die an die erste Kommunikationsvorrichtung übermittelten medizinischen Daten und/oder Dokumente und/oder Kontaktinformationen enthält, mittels derer eine Kommunikationsverbindung zu der ersten Kommunikationsvorrichtung herstellbar ist.

Gemäß einer weiteren Ausführungsform ist die zweite Kommunikationsvorrichtung ein Mobiltelefon, wobei die weitere Nachricht eine Kurznachricht ist, und wobei die Kontaktinformationen eine Telefonnummer sowie einen Eingabebefehl aufweisen, wobei durch einen Anruf der Telefonnummer durch die zweite Kommunikationsvorrichtung und die Eingabe des Eingabebefehls eine automatische Anrufweiterleitung an die erste Kommunikationsvorrichtung aktivierbar ist.

Gemäß einer weiteren Ausführungsform ist die weitere Nachricht eine E-Mail, wobei die Kontaktinformationen eine Telefonnummer, einen Eingabebefehl sowie einen Hinweis aufweisen, dass Identifikationsdaten, insbesondere die Identifikationsdaten des ersten Benutzers, eingegeben werden müssen, um eine automatische Anrufweiterleitung an die erste Kommunikationsvorrichtung zu aktivieren, wobei nach Anruf der Telefonnummer und Eingabe des Eingabebefehls und der Identifikationsdaten eine automatische Anrufweiterleitung an die erste Kommunikationsvorrichtung aktivierbar ist.

Insbesondere ist die Recheneinheit dazu konfiguriert, sowohl eine Kurznachricht als auch eine E-Mail mit den Kontaktinformationen an die zweite Kommunikationsvorrichtung zu übermitteln, so dass ein automatischer Rückruf wahlweise direkt von der zweiten Kommunikationsvorrichtung oder von einem anderen Gerät initiierbar ist.

Gemäß einer weiteren Ausführungsform ist die Recheneinheit dazu ausgebildet, für den Fall, dass nach Übermittlung der Textnachricht mit der Aufforderung zu der Bestätigungseingabe an die zweite Kommunikationsvorrichtung innerhalb einer vordefinierten Zeitspanne keine Bestätigungseingabe erfolgt (und somit auch keine Bestätigungsnachricht von der zweiten Kommunikationsvorrichtung versendet wird), mindestens eine weitere Nachricht (z.B. eine Textnachricht), insbesondere von der Recheneinheit, an die zweite Kommunikationsvorrichtung zu übermitteln, wobei insbesondere die mindestens eine weitere Nachricht die an die erste Kommunikationsvorrichtung übermittelten medizinischen Daten und/oder Dokumente und/oder Kontaktinformationen enthält, mittels derer eine Kommunikationsverbindung zu der ersten Kommunikationsvorrichtung herstellbar ist.

Gemäß einer weiteren Ausführungsform ist die Recheneinheit dazu konfiguriert, bei Übereinstimmung der empfangenen Identifikationsdaten mit den gespeicherten Identifikationsdaten automatisch einen Sprachanruf oder Videoanruf zwischen der ersten Kommunikationsvorrichtung und der zweiten Kommunikationsvorrichtung zu initiieren, wobei die Recheneinheit insbesondere für den Fall, dass der Sprachanruf oder Videoanruf zwischen der ersten Kommunikationsvorrichtung und der zweiten Kommunikationsvorrichtung zustande kommt, dazu konfiguriert ist, automatisch eine Textnachricht an die zweite Kommunikationsvorrichtung zu übermitteln, wobei die Textnachricht die Aufforderung zu der Bestätigungseingabe enthält, und wobei die Recheneinheit dazu konfiguriert ist, bei Erhalt der Bestätigungseingabe die Bestätigungsnachricht automatisch zu empfangen.

Gemäß einer weiteren Ausführungsform ist die Recheneinheit dazu konfiguriert, nach Abschluss des Sprach- oder Videoanrufs eine Textnachricht mit einem Teil der medizinischen Daten und/oder Dokumente des ersten Benutzers an die erste Kommunikationsvorrichtung zu übermitteln.

Gemäß einer weiteren Ausführungsform ist die Recheneinheit dazu konfiguriert, für den Fall, dass der Sprachanruf oder Videoanruf zwischen der ersten Kommunikationsvorrichtung und der zweiten Kommunikationsvorrichtung nicht zustande kommt, automatisch eine Textnachricht an die zweite Kommunikationsvorrichtung zu übermitteln, wobei die Textnachricht Kontaktinformationen enthält, mittels derer eine Kommunikationsverbindung zu der ersten Kommunikationsvorrichtung herstellbar ist.

Insbesondere umfassen die Kontaktinformationen Kontaktdaten, insbesondere eine Telefonnummer, (insbesondere des Dienstleisters) und einen Eingabebefehl, durch dessen Eingabe durch einen Anruf bei der Telefonnummer eine automatische Anrufweiterleitung an die erste Kommunikationsvorrichtung aktivierbar ist.

Gemäß einer weiteren Ausführungsform ist die zweite Kommunikationsvorrichtung ein Mobiltelefon, wobei die Recheneinheit dazu konfiguriert ist, nach Nicht-Zustandekommen des Sprachanrufs oder der Videoverbindung automatisch eine Kurznachricht an die zweite Kommunikationsvorrichtung zu übermitteln, und wobei die Kontaktinformationen eine Telefonnummer sowie einen Eingabebefehl aufweisen, wobei durch einen Anruf der Telefonnummer durch die zweite Kommunikationsvorrichtung und die Eingabe des Eingabebefehls eine automatische Anrufweiterleitung an die erste Kommunikationsvorrichtung aktivierbar ist.

Gemäß einer weiteren Ausführungsform ist die Recheneinheit dazu konfiguriert, die nach Nicht-Zustandekommen des Sprachanrufs oder der Videoverbindung automatisch eine E-Mail an die zweite Kommunikationsvorrichtung zu übermitteln, wobei die Kontaktinformationen eine Telefonnummer, einen Eingabebefehl sowie einen Hinweis umfassen, dass Identifikationsdaten, insbesondere die Identifikationsdaten des ersten Benutzers, eingegeben werden müssen, um eine automatische Anrufweiterleitung an die erste Kommunikationsvorrichtung zu aktivieren, wobei nach Anruf der Telefonnummer und Eingabe des Eingabebefehls und der Identifikationsdaten eine automatische Anrufweiterleitung an die erste Kommunikationsvorrichtung aktivierbar ist.

Insbesondere ist die Recheneinheit dazu konfiguriert, sowohl eine Kurznachricht als auch eine E-Mail mit den Kontaktinformationen an die zweite Kommunikationsvorrichtung zu übermitteln, so dass ein automatischer Rückruf wahlweise direkt von der zweiten Kommunikationsvorrichtung oder von einem anderen Gerät initiierbar ist.

Gemäß einer weiteren Ausführungsform ist die Recheneinheit dazu konfiguriert, für den Fall, dass der Sprachanruf oder Videoanruf zwischen der ersten Kommunikationsvorrichtung und der zweiten Kommunikationsvorrichtung nicht zustande kommt, automatisch eine Textnachricht an die erste Kommunikationsvorrichtung zu übermitteln, wobei die Textnachricht einen Teil der abgerufenen medizinischen Daten und/oder Dokumente des ersten Benutzers enthält.

Gemäß einer weiteren Ausführungsform ist die Recheneinheit dazu konfiguriert, die Identifikationsdaten des ersten Benutzers mittels eines Sprachanrufs oder eines Videoanrufs von der ersten Kommunikationsvorrichtung zu empfangen.

Gemäß einer weiteren Ausführungsform ist die Recheneinheit dazu konfiguriert, zumindest der Teil der abgerufenen medizinischen Daten und/oder Dokumente mittels einer Sprachnachricht automatisch an die erste Kommunikationsvorrichtung zu übermitteln.

Insbesondere umfassen die medizinischen Daten und/oder Dokumente persönliche Daten des ersten Benutzers, wobei insbesondere die persönlichen Daten ein Geburtsdatum des ersten Benutzers, ein Geschlecht des ersten Benutzers, eine Körpergröße des ersten Benutzers, ein Körpergewicht des ersten Benutzers, Informationen über eine Augenfarbe des ersten Benutzers, Informationen über eine Blutgruppe des ersten Benutzers, Informationen über das Vorhandensein mindestens einer Allergie des ersten Benutzers, Informationen über das Vorhandensein mindestens einer Krankheit des ersten Benutzers, Informationen über mindestens ein Medikament, welches der erste Benutzer einnimmt und/oder Informationen über mindestens eine an dem ersten Benutzer durchgeführte Operation umfassen.

Gemäß einer weiteren Ausführungsform ist die Recheneinheit dazu konfiguriert, nach Abschluss des Sprach- oder Videoanrufs eine Textnachricht mit einem Teil der medizinischen Daten und/oder Dokumente des ersten Benutzers an die erste Kommunikationsvorrichtung zu übermitteln.

Insbesondere umfassen die medizinischen Daten und/oder Dokumente mindestens ein medizinisches Dokument des ersten Benutzers, wobei insbesondere das medizinische Dokument eine Vorsorgevollmacht, eine ärztliche Notfallanordnung, eine Patientenverfügung, ein Organspendeausweis, ein Impfpass und/oder ein Allergiepass ist.

Ein dritter Aspekt der Erfindung betrifft ein Computerprogramm, umfassend Befehle, die bei der Ausführung des Computerprogramms durch einen Computer diesen veranlassen, das Verfahren nach dem ersten Aspekt der Erfindung auszuführen.

Gemäß einer Ausführungsform weist das Computerprogramm ein Empfangsmodul zum automatischen Empfangen der Identifikationsdaten des ersten Benutzers von der ersten Kommunikationsvorrichtung auf.

Gemäß einer weiteren Ausführungsform weist das Computerprogramm ein Vergleichsmodul zum automatischen Vergleichen der empfangenen Identifikationsdaten mit gespeicherten Identifikationsdaten auf.

Gemäß einer weiteren Ausführungsform weist das Computerprogramm ein Abrufmodul zum automatischen Abrufen der gespeicherten medizinischen Daten und/oder Dokumente des ersten Benutzers bei Übereinstimmung der empfangenen Identifikationsdaten mit den gespeicherten Identifikationsdaten auf.

Gemäß einer weiteren Ausführungsform weist das Computerprogramm ein Übermittlungsmodul zum automatischen Übermitteln zumindest eines Teils der abgerufenen medizinischen Daten und/oder Dokumente an die erste Kommunikationsvorrichtung und/oder zum automatischen Übermitteln einer Benachrichtigung an die zweite Kommunikationsvorrichtung auf.

Gemäß einer weiteren Ausführungsform ist das Übermittlungsmodul dazu konfiguriert, nach Übermittlung einer Bestätigungsnachricht von der zweiten Kommunikationsvorrichtung, weitere medizinische Daten und/oder Dokumente, insbesondere die vollständigen medizinischen Daten und/oder Dokumente, des ersten Benutzers automatisch an die erste Kommunikationsvorrichtung zu übermitteln.

Gemäß einer weiteren Ausführungsform ist das Übermittlungsmodul dazu konfiguriert, bei Übereinstimmung der empfangenen Identifikationsdaten mit den gespeicherten Identifikationsdaten automatisch eine Textnachricht an die zweite Kommunikationsvorrichtung zu übermitteln, wobei die Textnachricht eine Aufforderung zu einer Bestätigungseingabe enthält, wobei das Übermittlungsmodul weiterhin dazu konfiguriert ist, bei Erhalt der Bestätigungseingabe die Bestätigungsnachricht automatisch von der zweiten Kommunikationsvorrichtung zu empfangen.

Gemäß einer weiteren Ausführungsform ist das Übermittlungsmodul dazu konfiguriert, nach Übermittlung der ersten Bestätigungsnachricht die weiteren medizinischen Daten und/oder Dokumente des ersten Benutzers dadurch an die erste Kommunikationsvorrichtung zu übermitteln, dass automatisch eine Textnachricht an die erste Kommunikationsvorrichtung gesendet wird, wobei die Textnachricht einen Dateianhang mit mindestens einer Datei, welche die weiteren medizinischen Daten und/oder Dokumente enthält, oder einen Link zum Abrufen der weiteren medizinischen Daten und/oder Dokumente enthält.

Gemäß einer weiteren Ausführungsform ist das Übermittlungsmodul dazu konfiguriert, nach Übermittlung der Bestätigungsnachricht mindestens eine weitere Nachricht an die zweite Kommunikationsvorrichtung zu übermitteln, wobei insbesondere die weitere Nachricht die an die erste Kommunikationsvorrichtung übermittelten medizinischen Daten und/oder Dokumente und/oder Kontaktinformationen enthält, mittels derer eine Kommunikationsverbindung zu der ersten Kommunikationsvorrichtung herstellbar ist.

Gemäß einer weiteren Ausführungsform weist das Computerprogramm ein Verbindungsmodul zu automatischen Initiieren eines Sprachanrufs oder Videoanrufs zwischen der ersten Kommunikationsvorrichtung und der zweiten Kommunikationsvorrichtung bei Übereinstimmung der empfangenen Identifikationsdaten mit den gespeicherten Identifikationsdaten auf, wobei insbesondere das Übermittlungsmodul dazu konfiguriert ist, für den Fall, dass der Sprachanruf oder Videoanruf zwischen der ersten Kommunikationsvorrichtung und der zweiten Kommunikationsvorrichtung zustande kommt, automatisch eine Textnachricht an die zweite Kommunikationsvorrichtung zu übermitteln, wobei die Textnachricht die Aufforderung zu der Bestätigungseingabe enthält, und bei Erhalt der Bestätigungseingabe die Bestätigungsnachricht automatisch zu empfangen.

Gemäß einer weiteren Ausführungsform ist das Übermittlungsmodul dazu konfiguriert, für den Fall, dass der Sprachanruf oder Videoanruf zwischen der ersten Kommunikationsvorrichtung und der zweiten Kommunikationsvorrichtung nicht zustande kommt, automatisch eine Textnachricht an die zweite Kommunikationsvorrichtung zu übermitteln, wobei die Textnachricht Kontaktinformationen enthält, mittels derer eine Kommunikationsverbindung zu der ersten Kommunikationsvorrichtung herstellbar ist.

Gemäß einer weiteren Ausführungsform ist die zweite Kommunikationsvorrichtung ein Mobiltelefon, wobei das Übermittlungsmodul dazu konfiguriert ist, automatisch eine Kurznachricht an die zweite Kommunikationsvorrichtung zu übermitteln, wobei die Kontaktinformationen eine Telefonnummer sowie einen Eingabebefehl aufweisen, wobei durch einen Anruf der Telefonnummer durch die zweite Kommunikationsvorrichtung und die Eingabe des Eingabebefehls eine automatische Anrufweiterleitung an die erste Kommunikationsvorrichtung aktivierbar ist.

Gemäß einer weiteren Ausführungsform ist das Übermittlungsmodul dazu konfiguriert, automatisch eine E-Mail an die zweite Kommunikationsvorrichtung zu übermitteln, wobei die Kontaktinformationen eine Telefonnummer, einen Eingabebefehl sowie einen Hinweis aufweisen, dass Identifikationsdaten, insbesondere die Identifikationsdaten des ersten Benutzers, eingegeben werden müssen, um eine automatische Anrufweiterleitung an die erste Kommunikationsvorrichtung zu aktivieren, wobei nach Anruf der Telefonnummer und Eingabe des Eingabebefehls und der Identifikationsdaten eine automatische Anrufweiterleitung an die erste Kommunikationsvorrichtung aktivierbar ist.

Insbesondere ist das Übermittlungsmodul dazu konfiguriert, sowohl eine Kurznachricht als auch eine E-Mail mit den Kontaktinformationen an die zweite Kommunikationsvorrichtung zu übermitteln, so dass ein automatischer Rückruf wahlweise direkt von der zweiten Kommunikationsvorrichtung oder von einem anderen Gerät initiierbar ist.

Gemäß einer weiteren Ausführungsform ist das Übermittlungsmodul dazu konfiguriert, für den Fall, dass nach Übermittlung der Textnachricht mit der Aufforderung zu der Bestätigungseingabe an die zweite Kommunikationsvorrichtung innerhalb einer vordefinierten Zeitspanne (z.B. 30 Sekunden) keine Bestätigungseingabe erfolgt, automatisch mindestens eine weitere Nachricht an die zweite Kommunikationsvorrichtung zu übermitteln, wobei insbesondere die mindestens eine weitere Nachricht die an die erste Kommunikationsvorrichtung übermittelten medizinischen Daten und/oder Dokumente und/oder Kontaktinformationen enthält, mittels derer eine Kommunikationsverbindung zu der ersten Kommunikationsvorrichtung herstellbar ist.

Gemäß einer weiteren Ausführungsform ist das Übermittlungsmodul dazu konfiguriert, für den Fall, dass der Sprachanruf oder Videoanruf zwischen der ersten Kommunikationsvorrichtung und der zweiten Kommunikationsvorrichtung nicht zustande kommt, automatisch eine Textnachricht an die erste Kommunikationsvorrichtung zu übermitteln wird, wobei die Textnachricht einen Teil der abgerufenen medizinischen Daten und/oder Dokumente des ersten Benutzers enthält.

Gemäß einer weiteren Ausführungsform ist das Empfangsmodul dazu konfiguriert, die Identifikationsdaten des ersten Benutzers mittels eines Sprachanrufs oder eines Videoanrufs von der ersten Kommunikationsvorrichtung zu empfangen.

Gemäß einer weiteren Ausführungsform ist das Übermittlungsmodul dazu konfiguriert, zumindest den Teil der abgerufenen medizinischen Daten und/oder Dokumente mittels einer Sprachnachricht automatisch an die erste Kommunikationsvorrichtung zu übermitteln.

Gemäß einer weiteren Ausführungsform ist das Übermittlungsmodul dazu konfiguriert, nach Abschluss des Sprach- oder Videoanrufs eine Textnachricht mit einem Teil der medizinischen Daten und/oder Dokumente an die erste Kommunikationsvorrichtung zu übermitteln.

Insbesondere umfassen die medizinischen Daten und/oder Dokumente persönliche Daten des ersten Benutzers, wobei insbesondere die persönlichen Daten ein Geburtsdatum des ersten Benutzers, ein Geschlecht des ersten Benutzers, eine Körpergröße des ersten Benutzers, ein Körpergewicht des ersten Benutzers, Informationen über eine Augenfarbe des ersten Benutzers, Informationen über eine Blutgruppe des ersten Benutzers, Informationen über das Vorhandensein mindestens einer Allergie des ersten Benutzers, Informationen über das Vorhandensein mindestens einer Krankheit des ersten Benutzers, Informationen über mindestens ein Medikament, welches der erste Benutzer einnimmt und/oder Informationen über mindestens eine an dem ersten Benutzer durchgeführte Operation umfassen.

Insbesondere umfassen die medizinischen Daten und/oder Dokumente mindestens ein medizinisches Dokument des ersten Benutzers, wobei insbesondere das medizinische Dokument eine Vorsorgevollmacht, eine ärztliche Notfallanordnung, eine Patientenverfügung, ein Organspendeausweis, ein Impfpass und/oder ein Allergiepass ist.

Soweit Alternativen einzelner trennbarer Merkmale in dieser Spezifikation als Ausführungsformen angegeben sind, versteht sich, dass die Alternativen frei kombinierbar sind, um weitere Ausführungsformen der hier offenbarten Erfindung zu bilden.

Weitere Einzelheiten und Vorteile der Erfindung werden durch die nachfolgende Beschreibung von Ausführungsbeispielen anhand von Figuren erläutert. Diese Ausführungsbeispiele sind nicht als beschränkend für den Schutzbereich der Erfindung zu verstehen.
- Fig. 1: zeigt schematisch eine Recheneinheit gemäß der Erfindung sowie eine erste und eine zweite Kommunikationsvorrichtung;
- Fig. 2: ist ein Fließdiagramm, welches Schritte eines beispielhaften erfindungsgemäßen Verfahrens zur Übertragung von medizinischen Daten und/oder Dokumenten darstellt;
- Fig. 3: ist ein weiteres Fließdiagramm, welches das Abrufen von medizinischen Daten und/oder Dokumenten nach einem Beispiel des erfindungsgemäßen Verfahrens schematisch darstellt.

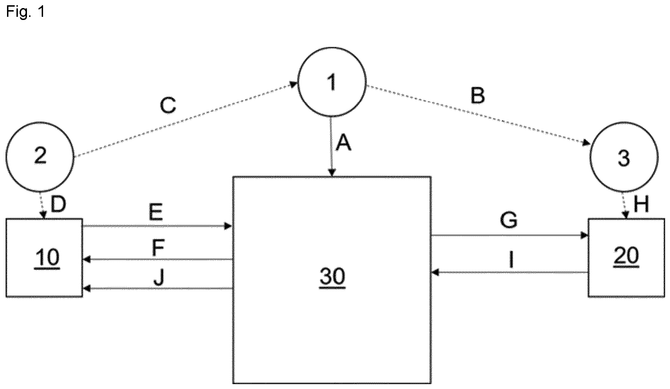

Fig. 1 zeigt schematisch die Handlungen der Benutzer bei dem erfindungsgemäßen Verfahren und Ihre Interaktionen mit den bei dem Verfahren verwendeten Vorrichtungen:
Ein erster Benutzer 1 hinterlegt in Schritt A medizinische Daten und Dokumente auf dem Speicher einer Recheneinheit 30, z.B. einem Server eines Dienstleisters, der die medizinischen Daten und Dokumente verwaltet. In Schritt B legt der erste Benutzer weiterhin einen dritten Benutzer 3 fest, dessen Kontaktdaten mit dessen Einverständnis auf dem Speicher der Recheneinheit als Notfallkontakt hinterlegt werden.

Zu einem späteren Zeitpunkt erleidet der erste Benutzer 1 einen medizinischen Notfall, z.B. einen Unfall, einen Herzinfarkt oder einen Schlaganfall und wird von dem zweiten Benutzer 2 (medizinisches Personal wie Rettungssanitäter oder Arzt) medizinisch versorgt (Schritt C).

Der zweite Benutzer 2 findet z.B. auf der Krankenversicherungskarte des ersten Benutzers 1 einen Aufkleber vor, der mit Hinweisen versehen ist, dass medizinische Daten und Dokumente des ersten Benutzers 1 bei einem Dienstleister hinterlegt sind, und wie diese abgerufen werden können. Weiterhin können z.B. auf dem Aufkleber Kontaktdaten des Dienstleisters (z.B. eine Telefonnummer) und persönliche Identifikationsdaten (z.B. eine mehrstellige Identifikationsnummer) des ersten Benutzers 1 verzeichnet sein.

Auf Basis dieser Hinweise kontaktiert der zweite Benutzer 2 mit seiner ersten Kommunikationsvorrichtung 10 (z.B. Festnetztelefon, Mobiltelefon oder Computer) in Schritt D und E den Dienstleister über einen von der Recheneinheit 30 zur Verfügung gestellten automatischen Anrufdienst, wobei der zweite Benutzer 2 die persönlichen Identifikationsdaten des ersten Benutzers 1 eingibt, um diesen zu identifizieren. Nach erfolgter Verifizierung der Identifikationsdaten, d.h. automatischem Vergleich der übermittelten Identifikationsdaten mit gespeicherten Identifikationsdaten, übermittelt die Recheneinheit 30 automatisch die wichtigsten medizinischen Daten (einen Teil der auf der Recheneinheit 30 hinterlegten Daten) an die erste Kommunikationsvorrichtung 10 des zweiten Benutzers 2. Diese Daten können z.B. die Blutgruppe des ersten Benutzers 1 oder Informationen über Allergien des ersten Benutzers 1 enthalten.

Der zweite Benutzer 2 kann anschließend den ersten Benutzer 1 aufgrund der Kenntnis dieser medizinischen Daten optimal versorgen.

Nach Verifizierung der Identifikationsdaten wird weiterhin in Schritt G von der Recheneinheit 30 automatisch eine Benachrichtigung an eine zweite Kommunikationsvorrichtung 20 des dritten Benutzers 3 (Notfallkontakt) versendet.

Durch diese Benachrichtigung wird der dritte Benutzer 3 schnell auf den medizinischen Notfall des ersten Benutzers 1 aufmerksam gemacht und kann sich somit z.B. zu dem Krankenhaus oder der Arztpraxis begeben, in dem bzw. der der erste Benutzer 1 versorgt wird oder kann ggf. weitere Personen benachrichtigen.

Zusätzlich erhält der dritte Benutzer 3 mit der Benachrichtigung insbesondere eine Aufforderung, die Übermittlung weiterer medizinischer Daten bzw. Dokumente, insbesondere der vollständigen medizinischen Daten und Dokumente des ersten Benutzers 1 an der zweiten Benutzer 2 zu bestätigen.

Im Schritt I sendet der dritte Benutzer 3 mittels der zweiten Kommunikationsvorrichtung 20 eine entsprechende Bestätigungsnachricht an die Recheneinheit 30, woraufhin die Recheneinheit 30 automatisch die weiteren medizinischen Daten und Dokumente, insbesondere die vollständigen medizinischen Daten und Dokumente, des ersten Benutzers 1 an die erste Kommunikationsvorrichtung 10 des zweiten Benutzers 2 übermittelt (Schritt J).

Bei diesen weiteren medizinischen Daten und Dokumenten kann es sich z.B. um medizinische Dokumente wie Vorsorgevollmachten oder Patientenverfügungen handeln, welche nicht für die unmittelbare Versorgung des ersten Benutzers 1 essentiell sind, jedoch zu einem späteren Zeitpunkt der medizinischen Versorgung wichtig sein können.

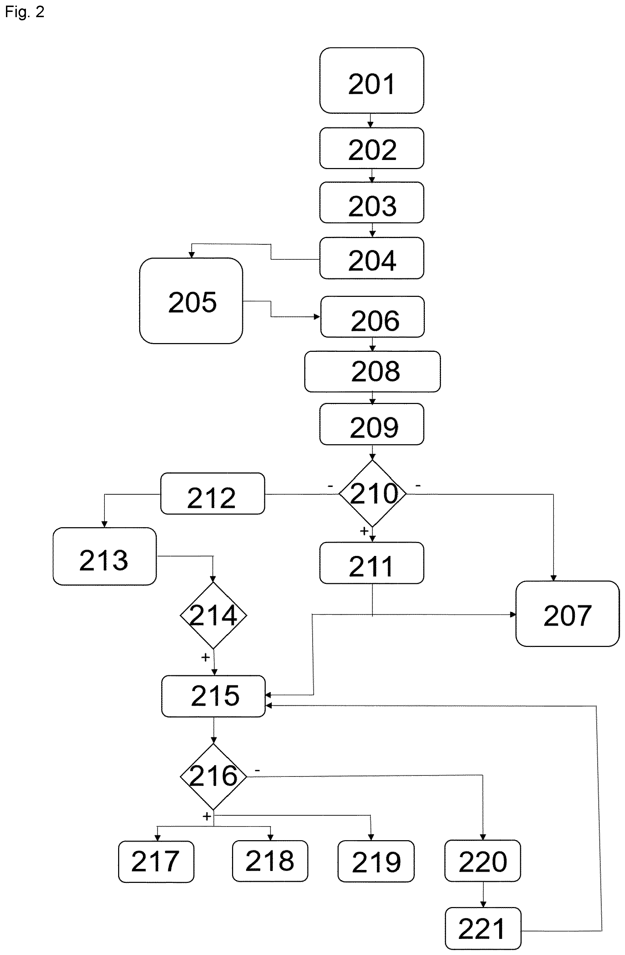

Fig. 2 ist ein Fließdiagramm einer beispielhaften Ausführungsform des erfindungsgemäßen Verfahrens.

In Schritt 201 findet der zweite Benutzer 2 (medizinisches Personal) die Notfallkontaktdaten des ersten Benutzers 1 vor und ruft in Schritt 202 mit der ersten Kommunikationsvorrichtung 10, z.B. mit seinem Mobiltelefon, den automatischen Anrufdienst des Betreibers an, welcher die medizinischen Daten des ersten Benutzers 1 hinterlegt hat. Nach einer entsprechenden Aufforderung 203 (z.B. einer automatischen Sprachansage) gibt dieser in Schritt 204 die Identifikationsdaten des ersten Benutzers 1 ein, z.B. über die Tastatur oder über eine Spracheingabe. In Schritt 205 generiert die Recheneinheit 30 eine weitere Sprachansage, welche den zweiten Benutzer informiert, dass in Kürze die wichtigsten medizinischen Daten des ersten Benutzers 1 an die erste Kommunikationsvorrichtung 10 übermittelt werden. Die wichtigsten medizinischen Daten werden z.B. in Schritt 206 per Sprachansage übermittelt. Anschließend generiert die Recheneinheit 30 eine weitere automatische Sprachansage, welche den zweiten Benutzer 2 informiert, dass er in Kürze über einen Sprach- oder Videoanruf automatisch mit dem dritten Benutzer 3 (hinterlegter Notfallkontakt) verbunden wird (Schritt 208). Im Schritt 209 versucht die Recheneinheit 30 eine solche Sprach- oder Videoverbindung zwischen der ersten Kommunikationsvorrichtung 10 des zweiten Benutzers 2 (medizinisches Personal) und der zweiten Kommunikationsvorrichtung 20 des dritten Benutzers 3 (Notfallkontakt) herzustellen.

In dem Sprach- oder Videoanruf können der zweite Benutzer 2 und der dritte Benutzer 3 Informationen austauschen. Z.B. kann der dritte Benutzer 3 anhand dieser Informationen verifizieren, dass es sich bei dem Anrufer wirklich um autorisiertes medizinisches Personal handelt.

Die Raute 210 in Fig. 2 symbolisiert eine Fallunterscheidung abhängig davon, ob der in Schritt 209 initiierte Sprach- oder Videoanruf zustande gekommen ist. Ist dies der Fall (+) wird in Schritt 211 überprüft, ob der Sprach- oder Videoanruf abgeschlossen ist. Falls ja, wird in Schritt 215 von der Recheneinheit 30 automatisch eine Textnachricht an die zweite Kommunikationsvorrichtung 20 des dritten Benutzers 3 (Notfallkontakt) versendet, die eine Aufforderung enthält, die Übermittlung weiterer medizinischer Daten und/oder Dokumente, bzw. der vollständigen medizinischen Daten und/oder Dokumente des ersten Benutzers 1 zu bestätigen.

Zusätzlich wird eine Textnachricht mit den wichtigsten medizinischen Daten und/oder Dokumente des ersten Benutzers 1 an die erste Kommunikationsvorrichtung 10 des zweiten Benutzers 2 versendet (Schritt 207), insbesondere mit denjenigen medizinischen Daten und/oder Dokumente, die schon im Schritt 206 per Sprachansage übermittelt wurden.

Die Raute 216 in Fig. 2 symbolisiert die Entscheidung, ob der dritte Benutzer 3 mit der zweiten Kommunikationsvorrichtung 20 eine Bestätigungsnachricht, z.B. eine Textnachricht, versendet, welche die Übermittlung der weiteren oder vollständigen medizinischen Daten und/oder Dokumente des ersten Benutzers 1 an die erste Kommunikationsvorrichtung 10 des zweiten Benutzers 2 bestätigt (+) oder nicht (-).

Im Fall einer Bestätigung (+) werden in Schritt 217 eine Textnachricht an die erste Kommunikationsvorrichtung 10 mit zusätzlicher Information sowie mit einem Link gesendet, der es ermöglicht, die weiteren oder vollständigen medizinischen Daten und Dokumente des ersten Benutzers 1 abzurufen, z.B. herunterzuladen.

Gleichzeitig wird im Schritt 218 von der Recheneinheit 30 automatisch eine E-Mail an den dritten Benutzer 3 versendet, welche eine Bestätigung sowie die an den zweiten Benutzer 2 übermittelten Daten und ggf. Dokumente (bzw. einen Link, über den die Daten bzw. Dokumente abrufbar sind) enthält, und im Schritt 219 werden von der Recheneinheit 30 automatisch eine kurze Textnachricht an den dritten Benutzer 3 versendet, wobei die kurze Textnachricht Kontaktinformationen enthält, mittels derer der zweite Benutzer 2 kontaktiert werden kann. Bei den Kontaktinformationen handelt es sich insbesondere um die Telefonnummer des Dienstleisters und einen Eingabebefehl, dessen Eingabe (z.B. Spracheingabe oder Eingabe über die Tasten/Nummernfelder des Telefons) eine automatische Anrufweiterleitung aktiviert wird. Mithilfe dieser Kontaktinformationen kann der dritte Benutzer 3 (Notfallkontakt) den zweiten Benutzer 2 (medizinisches Personal) direkt kontaktieren, um z.B. Rückfragen zu stellen.

Wenn im Schritt 216 nach einer vorgegebenen Zeitspanne, z.B. 30 Sekunden, keine Bestätigung erfolgt, also z.B. falls keine Antwort "ja" sondern eine explizite Antwort "nein" (oder entsprechende Eingaben), gar keine Antwort oder eine andere Antwort übermittelt wird, (-) werden in Schritt 220 eine Textnachricht und eine E-Mail an die zweite Kommunikationsvorrichtung 20 versendet, die Kontaktinformationen enthält, mittels derer der zweite Benutzer 2 kontaktiert werden kann. Kommt daraufhin ein Rückruf zwischen dem dritten Benutzer 3 und dem zweiten Benutzer 2 zustande, so wird erneut die Textnachricht mit der Aufforderung zur Bestätigungseingabe für die Übermittlung der vollständigen medizinischen Daten und Dokumente an die zweite Kommunikationsvorrichtung gesendet (Schritt 215)

Wird in Schritt 210 festgestellt, dass der Sprach- oder Videoanruf zwischen der ersten Kommunikationsvorrichtung 10 und der zweiten Kommunikationsvorrichtung 20 nicht zustande gekommen ist (Raute 210, mit "-" bezeichnete Pfeile und Box 212, die symbolisiert, dass keine Antwort des dritten Benutzers erhalten wurde), wird ebenfalls eine Textnachricht mit den wichtigsten medizinischen Daten und/oder Dokumente des ersten Benutzers 1, z.B. denselben Daten, die schon über die Sprachnachricht in Schritt 206 versendet wurden, an die erste Kommunikationsvorrichtung 10 des zweiten Benutzers 2 versendet (Schritt 207).

Zusätzlich werden in Schritt 213 eine kurze Textnachricht und eine E-Mail an den dritten Benutzer 3 (insbesondere an die zweite Kommunikationsvorrichtung 20) versendet, in welchen der dritte Benutzer 3 über den verpassten Sprach- oder Videoanruf informiert und zu einem Rückruf aufgefordert wird, wobei die entsprechenden Kontaktinformationen angegeben werden, mittels derer eine Kommunikationsverbindung zu der ersten Kommunikationsvorrichtung herstellbar ist.

Ruft der dritte Benutzer 3 dann zurück (Raute 214), fährt die Recheneinheit 30 mit dem automatischen Versenden einer Textnachricht an die zweite Kommunikationsvorrichtung 20 mit der Aufforderung zu der Bestätigungseingabe fort (Schritt 215, siehe oben).

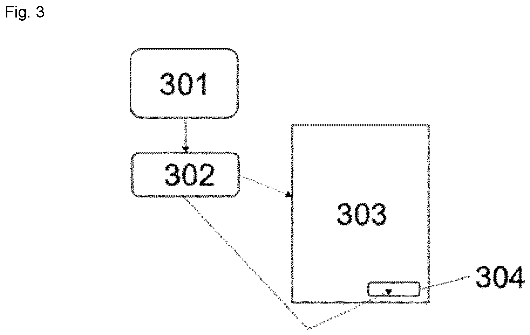

Fig. 3 zeigt weitere Schritte des Verfahrens, die sich an den oben beschriebenen Schritt 217 anschließen. Der zweite Benutzer 2 (medizinisches Personal) aktiviert in Schritt 301 den in Schritt 217 über die Textnachricht erhaltenen Link zu den weiteren bzw. vollständigen medizinischen Daten und Dokumenten des ersten Benutzers 1 (Patient) und öffnet dabei insbesondere in Schritt 302 eine Website 303 mit einem Downloadbutton 304, über welchen die weiteren oder vollständigen medizinischen Daten und Dokumente des ersten Benutzers 1 auf die erste Kommunikationsvorrichtung 10 heruntergeladen werden können.

## Patentansprüche

1. Verfahren zum Übermitteln von medizinischen Daten und/oder Dokumenten, wobei
- Identifikationsdaten eines ersten Benutzers (1) automatisch von einer ersten Kommunikationsvorrichtung (10) empfangen werden,
- die empfangenen Identifikationsdaten automatisch mit gespeicherten Identifikationsdaten verglichen werden,
und wobei bei Übereinstimmung der empfangenen Identifikationsdaten mit den gespeicherten Identifikationsdaten:
- gespeicherte medizinische Daten und/oder Dokumente des ersten Benutzers (1) automatisch abgerufen werden,
- zumindest ein Teil der abgerufenen medizinischen Daten und/oder Dokumente automatisch an die erste Kommunikationsvorrichtung (10) übermittelt wird, und
- automatisch eine Benachrichtigung an eine zweite Kommunikationsvorrichtung (20) übermittelt wird.

2. Verfahren nach Anspruch 1, wobei nach Übermittlung einer Bestätigungsnachricht von der zweiten Kommunikationsvorrichtung (20) weitere medizinische Daten und/oder Dokumente, insbesondere die vollständigen medizinischen Daten und/oder Dokumente, des ersten Benutzers (1) automatisch an die erste Kommunikationsvorrichtung (10) übermittelt werden.

3. Verfahren nach Anspruch 2, wobei bei Übereinstimmung der empfangenen Identifikationsdaten mit den gespeicherten Identifikationsdaten automatisch eine Textnachricht an die zweite Kommunikationsvorrichtung (20) übermittelt wird, wobei die Textnachricht eine Aufforderung zu einer Bestätigungseingabe enthält, wobei bei Erhalt der Bestätigungseingabe die Bestätigungsnachricht automatisch von der zweiten Kommunikationsvorrichtung (20) übermittelt wird.

4. Verfahren nach Anspruch 2 oder 3, wobei nach Übermittlung der Bestätigungsnachricht die weiteren medizinischen Daten und/oder Dokumente des ersten Benutzers (1) dadurch an die erste Kommunikationsvorrichtung (10) übermittelt werden, dass automatisch eine Textnachricht an die erste Kommunikationsvorrichtung (10) gesendet wird, wobei die Textnachricht einen Dateianhang mit mindestens einer Datei, welche die weiteren medizinischen Daten und/oder Dokumente enthält, oder einen Link zum Abrufen der weiteren medizinischen Daten und/oder Dokumente enthält.

5. Verfahren nach einem der Ansprüche 2 bis 4, wobei nach Übermittlung der Bestätigungsnachricht eine weitere Nachricht an die zweite Kommunikationsvorrichtung (20) übermittelt wird, wobei insbesondere die weitere Nachricht die an die erste Kommunikationsvorrichtung (10) übermittelten medizinischen Daten und/oder Dokumente und/oder Kontaktinformationen enthält, mittels derer eine Kommunikationsverbindung zu der ersten Kommunikationsvorrichtung (10) herstellbar ist.

6. Verfahren nach einem der vorhergehenden Ansprüche, wobei bei Übereinstimmung der empfangenen Identifikationsdaten mit den gespeicherten Identifikationsdaten automatisch ein Sprachanruf oder Videoanruf zwischen der ersten Kommunikationsvorrichtung (10) und der zweiten Kommunikationsvorrichtung (20) initiiert wird, wobei insbesondere für den Fall, dass der Sprachanruf oder Videoanruf zwischen der ersten Kommunikationsvorrichtung (10) und der zweiten Kommunikationsvorrichtung (20) zustande kommt, automatisch eine Textnachricht an die zweite Kommunikationsvorrichtung (20) übermittelt wird, wobei die Textnachricht die Aufforderung zu der Bestätigungseingabe enthält, wobei bei Erhalt der Bestätigungseingabe die Bestätigungsnachricht automatisch übermittelt wird.

7. Verfahren nach Anspruch 6, wobei für den Fall, dass der Sprachanruf oder Videoanruf zwischen der ersten Kommunikationsvorrichtung (10) und der zweiten Kommunikationsvorrichtung (20) nicht zustande kommt, automatisch eine Textnachricht an die zweite Kommunikationsvorrichtung (20) übermittelt wird, wobei die Textnachricht Kontaktinformationen enthält, mittels derer eine Kommunikationsverbindung zu der ersten Kommunikationsvorrichtung (10) herstellbar ist,

8. Verfahren nach Anspruch 6 oder 7, wobei für den Fall, dass der Sprachanruf oder Videoanruf zwischen der ersten Kommunikationsvorrichtung (10) und der zweiten Kommunikationsvorrichtung (20) nicht zustande kommt, automatisch eine Textnachricht an die erste Kommunikationsvorrichtung (10) übermittelt wird, wobei die Textnachricht einen Teil der abgerufenen medizinischen Daten und/oder Dokumente des ersten Benutzers (1) enthält.

9. Verfahren nach einem der vorhergehenden Ansprüche, wobei die Identifikationsdaten des ersten Benutzers (1) mittels eines Sprachanrufs oder eines Videoanrufs von der ersten Kommunikationsvorrichtung (10) empfangen werden.

10. Verfahren nach einem der vorhergehenden Ansprüche, wobei die Identifikationsdaten des ersten Benutzers (1) über eine Tastatur, eine Kamera, ein Mikrophon oder einen Fingerabdruckscanner der ersten Kommunikationsvorrichtung (10) oder über eine Tastatur, eine Kamera, ein Mikrophon oder einen Fingerabdruckscanner eines separaten Eingabegeräts erfasst werden.

11. Verfahren nach einem der vorhergehenden Ansprüche, wobei zumindest der Teil der abgerufenen medizinischen Daten und/oder Dokumente mittels einer Sprachnachricht automatisch an die erste Kommunikationsvorrichtung (10) übermittelt wird.

12. Verfahren nach einem der vorhergehenden Ansprüche, wobei die medizinischen Daten und/oder Dokumente persönliche Daten des ersten Benutzers (1) umfassen, wobei insbesondere die persönlichen Daten ein Geburtsdatum des ersten Benutzers (1), ein Geschlecht des ersten Benutzers (1), eine Körpergröße des ersten Benutzers (1), ein Körpergewicht des ersten Benutzers (1), Informationen über eine Augenfarbe des ersten Benutzers (1), Informationen über eine Blutgruppe des ersten Benutzers (1), Informationen über das Vorhandensein mindestens einer Allergie des ersten Benutzers (1), Informationen über das Vorhandensein mindestens einer Krankheit des ersten Benutzers (1), Informationen über mindestens ein Medikament, welches der erste Benutzer (1) einnimmt und/oder Informationen über mindestens eine an dem ersten Benutzer (1) durchgeführte Operation umfassen.

13. Verfahren nach einem der vorhergehenden Ansprüche, wobei die medizinischen Daten und/oder Dokumente mindestens ein medizinisches Dokument des ersten Benutzers (1) umfassen, wobei insbesondere das medizinische Dokument eine Vorsorgevollmacht, eine ärztliche Notfallanordnung, eine Patientenverfügung, ein Organspendeausweis, ein Impfpass und/oder ein Allergiepass ist.

14. Recheneinheit (30) zum Übermitteln von medizinischen Daten und/oder Dokumenten, insbesondere zur Durchführung eines Verfahrens nach einem der Ansprüche 1-13, wobei die Recheneinheit (30) dazu konfiguriert ist, Identifikationsdaten eines ersten Benutzers (1) von einer ersten Kommunikationsvorrichtung (10) automatisch zu empfangen, die empfangenen Identifikationsdaten automatisch mit gespeicherten Identifikationsdaten zu vergleichen, bei Übereinstimmung der empfangenen Identifikationsdaten mit den gespeicherten Identifikationsdaten automatisch gespeicherte medizinische Daten und/oder Dokumente des ersten Benutzers (1) abzurufen, zumindest einen Teil der abgerufenen medizinischen Daten und/oder Dokumente automatisch an die erste Kommunikationsvorrichtung (10) zu übermitteln und automatisch eine Benachrichtigung an eine zweite Kommunikationsvorrichtung (20) zu übermitteln.

15. Computerprogramm, umfassend Befehle, die bei der Ausführung des Programms durch einen Computer diesen veranlassen, das Verfahren nach einem der Ansprüche 1-13 auszuführen.
